# EUROPEAN PATENT APPLICATION

(11) **EP 4 268 596 A1**
(43) Date of publication of application: **01.11.2023**
(21) Application number: 21909611.2
(22) Date of filing: 22.12.2021
(51) Int. Cl.: A01N 63/50, A01N 59/20, A01N 55/02, A41D 31/30, A41D 13/11, A61L 2/16, D06M 11/83, B82Y 30/00

(54) **COATING AGENT BASED ON A COPPER-NANOPARTICLE BIOHYBRID AND USE THEREOF AS A BIOCIDAL AGENT**

(30) Priority: 22.12.2020 ES 202031282
(71) Applicant: Consejo Superior De Investigaciones Científicas (CSIC), 28006 Madrid (ES); Universidad De Zaragoza, 50009 Zaragoza (ES); Fundación Agencia Aragonesa para la Investigación y el Desarrollo (ARAID), 50018 Zaragoza (ES); Fundación Instituto de Investigación Sanitaria Aragón (IIS Aragón), 50009 Zaragoza (ES); Instituto Aragonés de Ciencias de la Salud (IACS), 50009 Zargoza (ES)
(72) Inventor: PALOMO CARMONA, Jose Miguel, 28049 Madrid (ES); LOSADA GARCÍA, Noelia, 28049 Madrid (ES); ABIAN FRANCO, Olga, 50009 Zaragoza (ES); VELÁZQUEZ CAMPOY, Adrián, 50009 Zaragoza (ES); ORTEGA ALARCÓN, David, 50018 Zaragoza (ES); JIMÉNEZ ALESANCO, Ana, 50018 Zaragoza (ES); CEBALLOS LAITA, Laura, 50009 Zaragoza (ES)
(74) Representative: Pons
(86) International application number: PCT/ES2021/070918
(87) International publication number: WO 2022/136716

(57) **Abstract**

The present invention relates to: a hybrid material comprising a protein matrix and nanoparticles (NPs) of a type of Cu; a preparation method thereof; and the uses thereof. The material of the invention displays activity that inhibits SARS-CoV-2 proteins, which makes it usable as a biocidal agent and as an agent for coating and disinfecting materials selected from metal, paper, textiles and approved surgical face masks.

## Description

The present invention relates to a hybrid material comprising a protein matrix and nanoparticles (NPs) of a type of Cu; a preparation method thereof; and the uses thereof. The material of the invention exhibits activity that inhibits SARS-CoV-2 proteins, which makes it usable as a biocidal agent and as an agent for coating and disinfecting materials selected from metal, paper, textiles and approved surgical face masks.

### BACKGROUND OF THE INVENTION

COVID-19 has endangered more than 210 countries and territories causing widespread human morbidity and mortality, more than 25 million confirmed infections and disruption to our economy, as well as major disruptions in daily life around the world. The disease is transmitted through the virus, SARS-CoV-2 (severe acute respiratory syndrome), which is known to remain viable on some solids for periods of up to 1 week.

The virus appears to be highly infectious and an important mode of transmission is thought to be from an infected person releasing virus-filled droplets of fluid that can shrink due to evaporation and thus form an aerosol. Another occurs when a person comes into contact with a solid that is contaminated with the virus (a fomite). Disposable surgical masks can prevent respiratory droplets from entering the lungs. However, there are some limitations of the existing masks (Addi, R. A., Benxim, A., Cherkaoui, M. Easybreath Decathlon Mask: An Efficient Personal Protective Equipment (PPE) against COVID-19 in Africa. J. Clin. Exp. Inv. 2020, 11*)*. Therefore, an anti-COVID-19 material would be very interesting.

Worldwide, the use of nanomaterials in the biomedical field has attracted increasing interest from researchers due to its unique property of interacting with cells and tissues at the molecular level, with a high degree of specificity and improved efficacy in combating infectious diseases (Dahoumane, S. A., Jeffryes, C., Mechouet, M., Agathos, S.N. Biosynthesis of inorganic nanoparticles: a fresh look at the control of shape, size and composition. Bioengineering 2017, 4, 1-16). Its small size, design flexibility and a large surface-to-volume ratio make these materials widely used.

Copper and its compounds have been used as disinfectant agents for many centuries. Since the 19th century, the discovery of a causal link between diseases and pathogens has revolutionized modern medicine. Research focused on the development of antimicrobial agents, especially antibacterial agents, and many studies have been done on the antibacterial effects of metals like copper.

In 2008, the EPA officially recognized copper and its alloys as the first effective metallic antimicrobial agent (http://www.epa.gov/pesticides/factsheets/copper-alloyroducts.htm). They recognized its ability to kill 99.9% of pathogenic bacteria within 2 hours. Since then, rapid progress has been made in the bactericidal properties of the surface of copper, known as "death by contact", which allows pathogenic bacteria to be eliminated quickly.

However, up until now, it has not been shown that it has a biocidal capacity as an additive in coating agents applied in solution.

### DESCRIPTION OF THE INVENTION

In the present invention, nanomaterials of highly stable copper nanoparticles with the capacity to inhibit the SARS-CoV-2 virus (anticovid material) have been developed. It has been shown that these nanomaterials with biocidal activity can be used as coating additives on surfaces of a different nature, exhibiting a high versatility.

Among the different applications derived from this capacity of the hybrid material of the invention include its use on materials for personal protective equipment (PPE). In this case, it has been applied to approved polypropylene surgical masks and to 100% cotton fabric (from a laboratory coat). The adhesion of the coating has been highly efficient and stable through washing cycles of the same and exposed to high temperatures, which would allow it to be used safely and to be reused at least 4 times. These masks would exhibit an amount of copper of approximately 1 g/mask, this is 5500 µg/cm² (>10 the recommended amount to eliminate the SARS-CoV-2 virus).

This results in a considerable increase in individual protection against the virus, increasing efficiency, for example, of surgical masks or those that are commercially available in fabric, directly inactivating the virus.

On the other hand, the hybrid material of the invention has also been applied to metallic materials, like steel or iron, such that it can be used both as a coating material and as an additive for direct disinfection of contact surfaces, either handrails or knobs, for use, for example, in the public transport sector. The amount of coating agent in the disinfectant solution would be, on this occasion, 800-10000 ppm of the nanohybrid per liter of water or aqueous disinfectant solution.

In the same manner, the coating agent of the invention can be applied dissolved in a hydroalcoholic solution, in which case, the amount of nanohybrid would be between 50 and 100 ppm in a 50% ethanol solution.

Lastly, its capacity for adherence and stability on paper has also been demonstrated, which could be of interest for its use as disposable anticovid material.

In a first aspect, the invention relates to the non-therapeutic use of a hybrid material comprising:
- a protein matrix composed of an enzyme or protein, and
- spherical nanoparticles of types of copper independently selected from Cu₃(PO₄)₂, Cu₂O and Cu (0), where said nanoparticles are distributed in a monodisperse manner within the matrix, as a biocidal agent.

In a preferred embodiment, the protein matrix is selected from *Candida antarctica* lipase B, *Thermomyces lanuginosus* lipase, *Bacillus thermocatenulatus* lipase, human albumin protein and catalase.

In another preferred embodiment, the nanoparticles are Cu₃(PO₄)₂, with a single diameter size distribution of between 3±0.5 nm and 15±0.5 nm, more preferably of between 3±0.5 nm and 11±0.5 nm, more preferably of between 3±0.5 nm and 10±0.5 nm; and yet more preferably of between 3±0.5 nm and 5±0.5 nm.

In another preferred embodiment, the biocidal agent is used against a virus selected from the hepatitis C virus, SARS-CoV, MERS-CoV, and SARS-CoV-2 that causes Covid-19.

In another preferred embodiment, the biocidal agent is used against a bacterium; and more preferably against bacteria selected from *Escherichia coli* and *Bacillus subtilis.*

In another preferred embodiment, the hybrid material acts as a coating agent, preferably on surfaces selected from ceramic materials, polymeric materials, metals, paper, textile and masks.

In a preferred embodiment, the metal is iron or steel.

In another preferred embodiment, the textile material is cotton, more preferably 100% cotton.

In another preferred embodiment, the coating agent is used as an additive for direct disinfection (disinfectant), and more preferably the agent is added to a solution selected from sodium hypochlorite, hydrogen peroxide, isopropanol, ethanol, didecyldimethylammonium chloride, and hexadecyltrimethylammonium bromide; and more preferably it is added to a solution selected from 0.1% sodium hypochlorite, 3% hydrogen peroxide, 70% isopropanol, 50%, 70% and 96% ethanol, 1.75% didecyldimethylammonium chloride , and hexadecyltrimethylammonium bromide.

A second aspect of the invention relates to a composite material comprising:
a. a product composed of a material selected from ceramic material, polymeric material, metal, paper, textile or a mask; and
b. a coating comprising the hybrid material as described above.

In a preferred embodiment, the metal is iron or steel.

In another preferred embodiment, the textile material is cotton, more preferably 100% cotton.

A third aspect of the present invention relates to a method for obtaining the composite material as previously described, which comprises applying the coating of the invention dissolved in a hydroalcoholic solution on the total or partial surface of a product composed of ceramic material, polymeric material, metal, paper, textile or a mask.

In a preferred embodiment, the coating is applied dissolved in a hydroalcoholic solution with a concentration of between 50:50 and 20:80 (water:alcohol). In a more preferred embodiment, the concentration is 50:50 water:alcohol.

In another preferred embodiment, the alcohol is selected from ethanol, propanol, isopropanol, methanol, among others. In a yet more preferred embodiment, the solvent is ethanol.

In another preferred embodiment, the coating is applied at a temperature of between 20 and 25°C on the total or partial surface of the product surface (a).

In another preferred embodiment, the coating method includes, without being limited to, liquid or gel immersion, spraying or spray coating, vacuum deposition, premolding or casting and extrusion.

Another aspect of the invention relates to the use of the coating agent as defined above as an additive for direct disinfection.

In a more preferred embodiment, the coating agent is used in combination with other disinfectant agents selected from sodium hypochlorite, hydrogen peroxide, isopropanol, ethanol, didecyldimethylammonium chloride, and hexadecyltrimethylammonium bromide.

In a yet more preferred embodiment, the coating agent is added to a solution selected from 0.1% sodium hypochlorite, 3% hydrogen peroxide, 70% isopropanol, 50%, 70% and 96% ethanol, 1.75% didecyldimethylammonium chloride, and hexadecyltrimethylammonium bromide.

In a fifth aspect, the present invention relates to a hybrid material comprising:
- a protein matrix composed of an enzyme or protein selected from *Thermomyces lanuginosus* lipase, *Bacillus thermocatenulatus* lipase, human albumin protein and catalase, and
- spherical nanoparticles of types of copper independently selected from Cu₃(PO₄)₂, Cu₂O and Cu (0), where said nanoparticles are distributed in a monodisperse manner within the matrix.

In a preferred embodiment, the nanoparticles are Cu₃(PO₄)₂, with a single diameter size distribution of between 3±0.5 nm and 15±0.5 nm, more preferably of between 3±0.5 nm and 11±0.5 nm, and more preferably of between 3±0.5 nm and 10±0.5 nm.

In another aspect, the present invention relates to a method for obtaining the previously described hybrid material, characterized in that it comprises the following steps:
a) addition under stirring of the enzyme or protein selected from *Thermomyces lanuginosus* lipase, *Bacillus thermocatenulatus* lipase, human albumin protein and catalase to a buffer solution of pH between 7 and 10;
b) addition of a copper salt to the solution obtained in step a) at room temperature of between 20 and 25°C;
c) incubation of the solution obtained in step b) for a time between 14-20 hours; and
d) collecting, and optionally reducing the hybrid material obtained in the previous step,
e) washing, and optionally, drying the product obtained from step (d).

In a preferred embodiment, the buffer in step a) is a phosphate buffer solution at a pH of between 6.5 and 7.5, more preferably at pH 7.

In another preferred embodiment, the collected hybrid material (which has formed as a solid in solution) is dried by lyophilization in step e).

Preferably, the copper salt is Cu₂SO₄; and yet more preferably it is Cu₂SO₄·5H₂O.

In a preferred embodiment, 10 mg of copper salt are added in step b) per ml of buffer solution.

In a preferred embodiment, the incubation time in step c) is 16 hours. Incubation means that the mixture is left under stirring for the specified time.

In another aspect, the present invention relates to a disinfectant solution comprising the hybrid material as previously described dissolved in a medium selected from sodium hypochlorite, hydrogen peroxide, isopropanol, ethanol, didecyldimethylammonium chloride, and hexadecyltrimethylammonium bromide.

In a preferred embodiment, the solution is selected from 0.1% sodium hypochlorite, 3% hydrogen peroxide, 70% isopropanol, 50%, 70% and 96% ethanol, 1.75% didecyldimethylammonium chloride, and hexadecyltrimethylammonium bromide.

In the present invention, the terms "hybrid material", "biohybrid material", "bionanohybrid material" or simply "biohybrid" or "bionanohybrid" or "hybrid material comprising a protein matrix and copper nanoparticles" are used synonymously and refer to a material comprising a protein matrix selected from *Candida antarctica* lipase B, *Thermomyces lanuginosus* lipase, *Bacillus thermocatenulatus* lipase, human albumin protein and catalase (organic compound) and types of Cu (inorganic compound) selected from: Cu (0), Cu₂O, Cu₃(PO₄)₂, , in the form of spherical nanoparticles where: when the type of copper is Cu₃(PO₄)₂, the single diameter size distribution is of between 3±0.5 nm and 11±0.5 nm; when the type of copper is Cu₂O, the single diameter size distribution is of between 10 and 20 ±0.5 nm; and when the type of copper is Cu(0), the single diameter size distribution is of between 6 and 10±0.5 nm; where said nanoparticles are distributed in a monodisperse manner within the matrix avoiding aggregation.

In the present invention, the term "single diameter size distribution" refers to the size number of nanoparticles obtained in each synthesis, and which, in any case, does not exceed the variation of ±0.5 nm. The nanoparticles of the present invention are spherical or substantially spherical.

In the present invention, "distributed in a monodisperse manner" is understood to mean a distribution of the nanoparticles on the protein matrix in such manner that their aggregation is avoided.

In the present invention, the *Candida antarctica* lipase B (CALB) (GenBank reference number: CAA83122.1) is a non-specific lipase of *Candida antarctica* with a molecular weight of around 34 kDa and an isoelectric point of 6. *Candida antarctica* lipase B is available commercially, for example, through Novozymes under the name Lipozyme^{®} CALB.

In the present invention, the *Thermomyces lanuginosus* lipase (TLL) (GenBank reference number: AF054513) is a remarkably thermostable basophilic lipase commercially available as Lipozyme^{®} TL 100 L from Novozymes, with a molecular weight of 32 KDa and an isoelectric point of 4.4.

In the present invention, *Bacillus thermocatenulatus* lipase 2 (BTL) (GenBank reference number: AST00902.1) is a remarkably thermostable non-specific thermoalkalophilic lipase of *Bacillus thermocatenulatus* expressed in *E.coli*, with a molecular weight of 43 KDa and an isoelectric point of 7.2.

In the present invention, the human albumin protein (HSA) (GenBank reference number AAX63425) is a protein found in a large proportion in lymphocytes, being the main protein in the blood, and one of the most abundant in humans. It has a molecular weight of 67 kDa and an isoelectric point of 4.6.

In the present invention, catalase (CAT) (GenBank reference number CAK40252.1) is a multimeric enzyme expressed in *Aspergillus niger* and commercially available from Novozymes under the name Catazyme*^{®}* 25 L. It has an approximate molecular weight of 400 kDa and an isoelectric point of 6.8.

Unless defined otherwise, all the technical and scientific terms used in the present document have the same meaning that is commonly understood by those of ordinary skill in the art to which this invention belongs. Methods and materials similar or equivalent to those described herein may be used in the practice of the present invention.

Throughout the description and the claims, the word "comprises" and its variants do not intend to exclude other technical features, additives, components or steps. For those skilled in the art, other objects, advantages and features of the invention may be partially deduced from both the description and the practice of the invention. The following examples and figures are provided by way of illustration and are not intended to limit the present invention.

### DESCRIPTION OF THE FIGURES

**FIG. 1****.** Characterization of NanoCu. A) X-ray diffraction spectrum (XRD); B) TEM and HRTEM microscopy images; C) Particle size distribution.
**FIG. 2****.** Spectroscopic characterization of NanoCu. A) Far-UV Circular Dichroism spectrum. B) Near-UV Circular Dichroism spectrum. C) Fluorescence spectrum. The fluorescence measurement was performed using an excitation length of 280 nm, with excitation and emission bands of 5 nm. NanoCu (dashed), free protein (continuous).
**FIG. 3****.** Characterization of the NanoCu-2 biohybrid prepared in Example 1 of the present invention. A) XRD spectrum, B) TEM images.
**FIG. 4****.** Characterization of the NanoCu-3 biohybrid prepared in Example 1 of the present invention. A) XRD spectrum, B) TEM images.
**FIG. 5****.** Characterization of different Cu-enzyme biohybrids prepared in Example 1 of the present invention. (A) NanoCu-TLL. (B) NanoCu-BTL. (C) NanoCu-CAT. (D) NanoCu-HSA. I) TEM microscopy images; II) High resolution microscopy (HR)-TEM images; III) Nanoparticle size distribution.

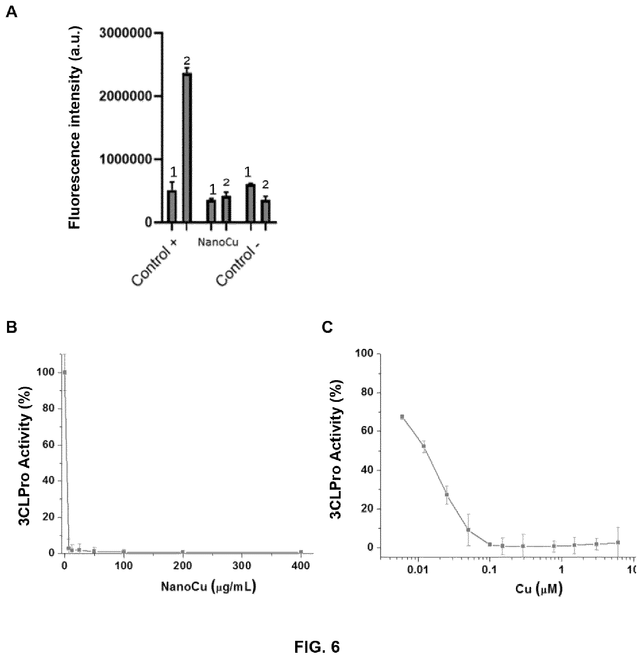

**FIG. 6****.** Functional characterization of NanoCu using a SARS-CoV-2 3CLpro protein activity assay in the presence of 30 µg/ml (A)* and increasing concentrations of NanoCu (B and C)**. The data is presented as a function of NanoCu concentration in µg/ml (B) and of Cu contained in the NanoCu in µM(C). Fluorescence resonance energy transfer (FRET) cleavage assay with 20 µm peptide substrate (Dabcyl)-KTSAVLQSGFRKME-(Edans)-NH2 (Biosyntan GmbH) in 100 µl. 0.2 µm 3CLpro in 50 mM sodium phosphate, NaCl 150 mM, at pH 7. FluoDia T70 Reader (λ_{excitation}= 380 nm; λₑₘᵢₛₛᵢₒₙ= 500 nm). * Endpoint assay: time 0 (1) and endpoint 20 min (2).** Continuous assay.
**FIG. 7****.** Functional characterization of NanoCu using a RayBio^{®} COVID-19 Spike-ACE2 (A)* binding assay in the presence of increasing concentrations of NanoCu in µg/ml (B). (A)* *Elisa test: Surface coating (ACE2); Preincubation 45 min 4 °C (Spike (1X) + Compounds-Cu (30 µg*/*ml)); Incubation 2.5 hours room temperature* / *overnight 4 °C (Spike + Compounds + ACE2); Antibody 1 hour room temperature (HRP conjugated antibody); Substrate 15 min room temperature (TMB); STOP (HCL 0.2 m). Measurement in spectrophotometer at* λ= *450 nm.*

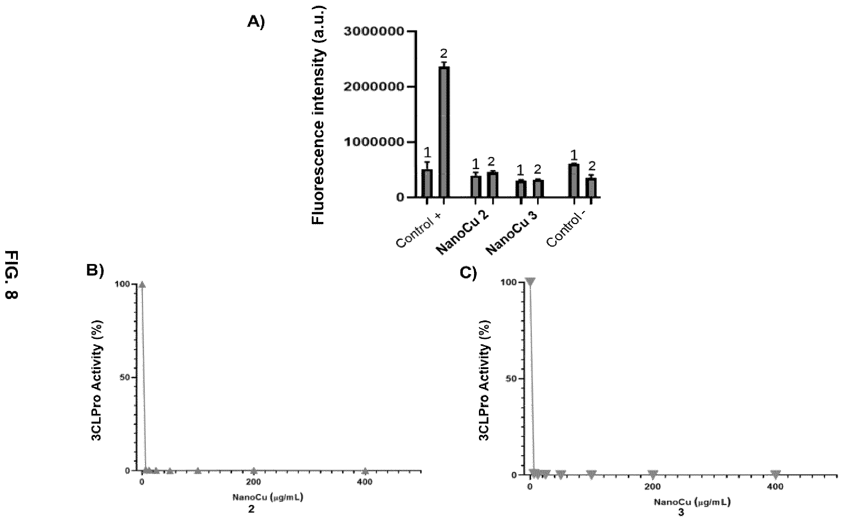

**FIG. 8****.** Functional characterization of other NanoCu materials using a SARS-CoV-2 3CLpro protein activity assay in the presence of 30 µg/ml (A)* and increasing concentrations of NanoCu (B and C)**. The data is presented as a function of NanoCu concentration in µg/ml for NanoCu-2 (NanoCu-2) (B) and NanoCu-3 (NanoCu-3) (C). Fluorescence resonance energy transfer (FRET) cleavage assay with 20 µm peptide substrate (Dabcyl)-KTSAVLQSGFRKME-(Edans)-NH2 (Biosyntan GmbH) in 100 µl. 0.2 µm 3CLpro in 50 mM sodium phosphate, NaCl 150 mM, at pH 7. FluoDia T70 Reader (λ_{excitation}= 380 nm; λₑₘᵢₛₛᵢₒₙ= 500 nm). Endpoint assay: time 0 (1) and endpoint 20 min (2).** Continuous assay Endpoint assay. ** Continuous assay.
**FIG. 9****.** Functional characterization of NanoCu-2 and NanoCu-3 using a RayBio^{®} COVID-19 Spike-ACE2* binding assay in the presence of increasing concentrations of material in µg/ml. NanoCu-2 (squares), NanoCu-3 (triangles). ** Elisa test: Surface coating (ACE2); Preincubation 45 min 4 °C (Spike (1X) + Compounds-Cu (30 µg*/*ml)); Incubation 2.5 hours room temperature* / *overnight 4 °C (Spike + Compounds + ACE2); Antibody 1 hour room temperature (HRP conjugated antibody); Substrate 15 min room temperature (TMB); STOP (HCL 0.2 m). Measurement in spectrophotometer at* λ= *450 nm.*
**FIG. 10****.** Functional characterization of new nanohybrids with other proteins by a SARS-CoV-2 3CLpro protein activity assay in the presence of increasing concentrations of nanobiohybrid in continuous assay. A) NanoCu-TLL, B) NanoCu-BTL, C) NanoCu-HSA, D) NanoCu-CAT. Fluorescence resonance energy transfer (FRET) cleavage assay with 20 µm peptide substrate (Dabcyl)-KTSAVLQSGFRKME-(Edans)-NH2 (Biosyntan GmbH) in 100 µl. 0.2 µm 3CLpro in 50 mM sodium phosphate, NaCl 150 mM, at pH 7. FluoDia T70 Reader (λ_{excitation}= 380 nm; λₑₘᵢₛₛᵢₒₙ= 500 nm).
**FIG. 11****.** 1) SEM microscopy images of the structure of the mask. 2) Image of the actual mask. A) Untreated surgical mask; B) Mask with NanoCu 50:50 Ethanol:water; C) Mask with NanoCu 70:30 Ethanol:water; D) Mask with NanoCu 80:20 Ethanol:water.
**FIG. 12****.** 1) SEM microscopy images of the mask structure with NanoCu coating in 50:50 ethanol:water; 2) Image of the actual mask. A) 1 g; B) 0.75 g; C) 0.5 g; D) 0.25 g; E) 0.12 g. All samples have been made in 45 cm².
**FIG. 13****.** Surface coating with NanoCu. A) 100% cotton fabric (10.5 cm²); B) Steel bar (6.9 cm²); C) Iron wrench (9 cm²); D) Paper (10.5 cm²). I) SEM images of the starting material; II) SEM images of the material with NanoCu; III) Physical sample of the material with NanoCu and dimensions (0.25 g of solid for all samples).
**FIG. 14****.** NanoCu stability against contact with disinfectants. A) Samples in Petri dishes with the different disinfectants. B) X-ray diffraction spectra (XRD) of the solids after the application of the disinfectant compared to the untreated solid. I) Multipurpose disinfectant (0.6% didecyldimethylammonium chloride); II) 3% H₂O₂; III) 0.1% bleach; IV) 70% ethanol; V) 96% ethanol, VI) 70% isopropanol, VII) 1.75% CTAB.
**FIG. 15****.** Catalytic efficiency of NanoCu. A) Samples treated with the different disinfectants compared with the fresh material and the dried material under the same conditions. B) NanoCu mixed directly with 0.1% bleach for 7 days. C) NanoCu mixed directly with 70% isopropanol for 7 days. D) NanoCu mixed directly with CTAB (1.75%) for 7 days. Catalytic efficiency was determined using the p-aminophenol oxidation assay. (C₂₂H₄₈ClN empirical formula of didecyldimethylammonium chloride).
**FIG. 16****.** SEM microscopy images of the structure of the mask with the NanoCu coating after washing with tap water (0.5 g of hybrid in 10.5 cm²). A) Room temperature, B) 50°C; 1) First wash, 2) Second wash, 3) Third wash.
**FIG. 17****.** SEM microscopy images of the structure of the mask with the NanoCu coating after washing with 50:50 ethanol:water (0.5 g of hybrid in 10.5 cm²). A) Room temperature, B) 50°C; 1) First wash, 2) Second wash, 3) Third wash.
**FIG. 18****.** SEM microscopy images of the structure of the mask with the NanoCu coating after washing with 70:30 Ethanol:water (0.5 g of hybrid in 10.5 cm²). A) Room temperature, B) 50°C; 1) First wash, 2) Second wash, 3) Third wash.
**FIG. 19****.** SEM microscopy images of the structure of the mask with the NanoCu coating after washing with 80:20 Ethanol:water (0.5 g of hybrid in 10.5 cm²). A) Room temperature, B) 50°C; 1) First wash, 2) Second wash, 3) Third wash.
**FIG. 20****.** SEM microscopy images of the NanoCu-coated mask against washes (0.12 g of hybrid in 10.5 cm²). A) Untreated sample; B) Samples at 40°C; C) Samples at 60°C. I) 30 min; II) 60 minutes; III) 120 min.
**FIG. 21****.** SEM microscopy images of NanoCu-coated 100% cotton fabric against washes (0.12 g of hybrid in 10.5 cm²). A) Untreated sample; B) Samples at 40°C; C) Samples at 60°C. I) 30 min; II) 60 minutes; III) 120 min.

### EXAMPLES

Next, the invention will be illustrated by means of assays carried out by the inventors that demonstrate the effectiveness of the product of the invention.

### Materials and Methods

Inductively coupled plasma-optical emission spectrometry (ICP-OES) was performed on an OPTIMA 2100 DV instrument (PerkinElmer, Waltham, MA, U. S.A.). X-ray diffraction (XRD) spectra were obtained using a D8 Advance Texture Analysis Diffractometer (Bruker, Billerica, MA, U. S.A.). With Cu Kα radiation. Scanning Electron Microscopy (SEM) images were taken in a TM-1000 microscope (Hitachi, Tokyo, Japan). To recover the biohybrids, a Biocen 22 R refrigerated centrifuge (Orto-Alresa, Ajalvir, Spain) was used.

### Circular dichroism measurements and fluorescence spectroscopy

The circular dichroism (CD) spectra of the protein and the hybrid were recorded in a Chirascan spectropolarimeter (Applied Photophysics) at 25 (±1)°C. Far UV spectra were recorded at wavelengths of between 190 and 260 nm in a 0.1 cm path length cuvette. Near UV spectra were recorded at wavelengths of between 250 and 310 nm in a 1 cm path length cuvette. Protein concentrations were 20 and 10 µm respectively in phosphate-buffered saline solution, pH 7.2 (PBS; bioMerieux). Regarding fluorescence measurements, they were performed in a Varian Cary Eclipse fluorescence spectrophotometer (Agilent Technologies) monitoring the intrinsic fluorescence of tryptophan in 2 µm of the hybrid solution, using an excitation wavelength of 280 nm, with excitation and emission bandwidths of 5 nm, and recording fluorescence emission spectra between 300 and 400 nm with a step of 1 nm. All spectroscopic measurements were performed in water.

### Detection of 3CLPro protein inhibition

The 3CLpro protein of the SARS-CoV-2 virus has protease activity and its inhibition can be monitored using a substrate in continuous or endpoint assays. Activity measurements have been made with a peptide that is a specific substrate of the protein and whose hydrolysis can be detected by FRET fluorescence.

If the 3CLpro protein comes into contact with the derivatives under study that are the object of this patent, in case of interaction between them and subsequent inhibition, we would observe a decrease in the detected fluorescence intensity (since the hydrolysis of the FRET substrate would not occur when the protease activity is inhibited).

Activity measurements have been carried out in two ways:

### 1. Continuous activity measurement test protocol:

The *in vitro* catalytic activity of 3CLpro was determined using a continuous fluorescence resonance energy transfer (FRET) assay with the peptide substrate (Dabcyl)-KTSAVLQSGFRKME-(Edans)-NH2 (Biosyntan GmbH). The enzyme was used at a final concentration of 0.2 µm in 50 mM sodium phosphate, NaCl 150 mM, at pH 7, and the enzymatic reaction was started by adding the substrate to a final concentration of 20 µm, in a final volume of 100 µl. Fluorescence was measured continuously in a FluoDia T70 microplate reader (Photon Technology International) for 60 minutes (excitation wavelength, 380 nm; emission wavelength, 500 nm). The enzymatic activity was quantified as the initial slope of the time evolution curve of the fluorescence signal. To study protease inhibition by compounds, they were added in concentrations ranging from 0 to 400 µg/ml, and the ratio of enzyme activity in the presence and absence of a given compound provides the percentage inhibition at each concentration.
2/ A fluorescence detection method at a fixed concentration of derivative and at two times (0 and 20 min), removing the derivative by centrifugation and stopping the reaction by heat inactivation of the protease. In this way, it is verified that there have been no interferences that may be due to the presence of the derivatives in the measurement of the fluorescence of the samples.

### 2. Endpoint activity measurement assay protocol:

Enzyme activity was determined using the same fluorescence resonance energy transfer (FRET) substrate, (Dabcyl)-KTSAVLQSGFRKME-(Edans)-NH₂ (Biosyntan, GmbH). To avoid the possibility of radiation scattering or attenuation by BIOG, an endpoint assay was carried out to separate the particles before the fluorescence measurement.

In summary, 550 µl of 3CLpro (0.2 µm final concentration) was mixed in assay buffer, containing 50 mM sodium phosphate pH 7, NaCl 150 mM, with 10.5 µl of compounds (final concentration 30 µg/ml).

Next, the reaction was started by adding the substrate to a final concentration of 20 µm, in a final volume of 700 µl. A starting aliquot was taken for each sample, leaving the rest of the solution for 20 minutes at room temperature. The enzymatic reaction was stopped at the two different time points (0 and 20 minutes) by heat treatment at 90°C for 5 minutes. Particles were removed by centrifugation for 3 minutes at 10000 rpm. The release of EDANS from the substrate was measured fluorimetrically from the supernatant in triplicate on a FluoDia T70 microplate reader (Photon Technology International, excitation wavelength, 380 nm; emission wavelength, 500 nm).

Enzyme inhibition was calculated by estimating the percentage of activity compared to two controls: the protein without any compound was taken as 100% activity, and 0% was estimated from residual fluorescence from a sample containing only assay buffer and substrate.

To check whether the compounds interfere with the detection of the reaction product, the same method was carried out on samples where the substrate was replaced by the free fluorophore of the substrate (EDANS) at the same concentration.

### Detection of inhibition of Spike-ACE2 interaction

The RayBio^{®} COVID-19 Spike-ACE2 Binding Assay Kit is a rapid, simple and sensitive method to characterize the binding affinity of the Spike-ACE2 complex and to assess the activity of potential inhibitors. This *in vitro* enzyme-detection immunoassay can measure numerous reagents and conditions simultaneously. We can determine if the derivatives under study can alter the interaction of the Spike protein of the SARS-CoV-2 virus and prevent its interaction with the human ACE2 protein that is immobilized on the surface. If the signal of the test after the development decreases after the contact of the derivatives with the Spike protein, it indicates that Spike's further interaction with ACE2 has been blocked.

### Example 1. Synthesis of the biohybrids (NanoCu, NanoCu-2, NanoCu-3, NanoCu-TLL, NanoCu-BTL, NanoCu-CAT, NanoCu-HSA)

### NanoCu

1.8 ml of commercial solution of *Candida antarctica* lipase (CALB) (18 mg protein) were added to 60 ml sodium phosphate buffer (0.1 m, pH 7) in a 250 ml glass bottle containing a small magnetic bar stirrer (2.5 cm x 0.5 cm; 360 rpm). Then, 600 mg of Cu₂SO₄ x 5H₂O (10 mg/ml) were added to the protein solution under stirring and the mixture continued to be stirred for 16 hours at room temperature. After the first 30 min of incubation, the solution turned a cloudy (turquoise) color that was preserved at all times. After 16 hours, the mixture was centrifuged at 8000 rpm for 10 min. (6 falcon tubes, 10 ml in each). Once the liquid in each falcon tube was removed, the solids obtained were washed, to remove possible remains of copper salt or protein, adding 10 ml of distilled water to each tube and stirring the mixture slightly with a spatula. Once mixed, they were centrifuged under the same conditions as before. The method was repeated two more times. Lastly, the supernatant was removed and, as a result, after joining all the solids obtained in the different falcon tubes, 3.5 g of wet solid were obtained. It was found that they corresponded to 350 mg of dry solid.

The characterization of NanoCu was performed by XRD analysis, ICP-OES, TEM, SEM, DC and fluorescence.

### NanoCu-2 and NanoCu-3

1.8 ml (18 mg of protein) of commercial solution of *Candida antarctica* lipase (CALB) was added to a 60 ml 0.1 m buffer (sodium phosphate pH 7 for NanoCu-2 or sodium bicarbonate pH =10 for NanoCu-3) in a 250 ml glass bottle containing a small magnetic bar stirrer. Then, 600 mg of Cu₂SO₄ x 5H₂O (10 mg/ml) were added to the protein solution and maintained for 16 hours. After the first 30 min of incubation, the solution turned cloudy (turquoise). After 16 hours, 6 ml of NaBH₄ (300 mg) (1.2 M) aqueous solution was added to the cloudy solution (3 ml twice) obtaining a final concentration of 0.12 M sodium borohydride in the mixture. The solution quickly turned black and the mixture was reduced for 30 minutes. After incubation, in all cases, the mixture was centrifuged at 8000 rpm for 5 min (10 ml per falcon tube). The generated pellet was resuspended in 15 ml of water. It was centrifuged again at 8000 rpm for 5 min and the supernatant was removed. The process was repeated two more times. Lastly, the supernatant was removed and the pellet from each falcon was resuspended in 2 ml of water, all solutions were collected in a round bottom flask, they were frozen with liquid nitrogen and lyophilized for 16 hours. Then, 150 mg of the so-called NanoCu-2 and NanoCu-3 were obtained, respectively.

The characterization of the different Cu biohybrids was performed by XRD analysis, ICP-OES, TEM and SEM.

### NanoCu-TLL, NanoCu-BTL, NanoCu-CAT, NanoCu-HSA

The synthesis of these biohybrids has been carried out in an aqueous medium by adding *Thermomyces lanuginosus* lipase (TLL), *Bacillus thermocatenulatus* lipase (BTL), catalase (CAT) or human albumin (HSA) to 60 ml of an aqueous solution containing 100 mM sodium phosphate buffer (pH 7). In each case the amounts of enzyme added were: 0.75 ml of commercial TLL solution (24 mg/ml), 0.5 ml of CAT (32 mg/ml and 20 mg of commercial HSA, respectively.

In the case of BTL, 20 ml (0.4 mg/ml solution containing 0.5% (w/v) Triton X-100 from the purification step) was added corresponding to an enzyme concentration of 0.13 mg/ml at 40 ml of phosphate buffer pH7.

Then, in each case, 600 mg of copper (II) sulfate pentahydrate [Cu₂SO₄ x 5H₂O] (10 mg/ml) was added to the protein solution under stirring and the mixture continued to be stirred for 16 hours at room temperature.

After incubation, the solid obtained was centrifuged, washed with the NanoCu protocol and lyophilized for 16 hours to obtain the heterogeneous NanoCu-TLL biohybrids, NanoCu-BTL, NanoCu-CAT, and NanoCu-HSA, respectively. These biohybrids were characterized by different analysis techniques such as XRD, ICP-OES, TEM and SEM.

### Example 2. Characterization of the biohybrids (NanoCu, NanoCu-2 and NanoCu-3)

### NanoCu

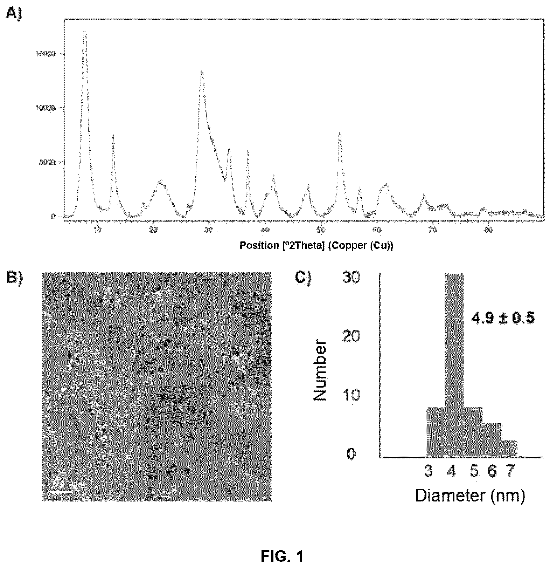

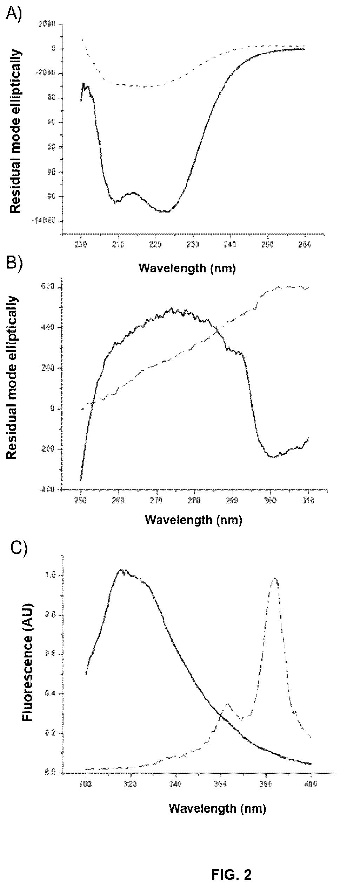

NanoCu was structurally characterized by different techniques such as X-ray diffraction (XRD) (to determine its metallic type), transmission electron microscopy (TEM) to determine the size of the copper nanoparticles that make up the material, as well as circular dichroism (CD) and fluorescence (F) assays to confirm the coordination and presence of types of copper in the protein matrix. XRD analysis showed that the type of copper in NanoCu was Cu₃(PO₄)₂ (Figure 1A). TEM analysis showed the formation of nanoparticles of approximately 4.9 nm in size (Figure 1B-C). The copper content of the material was determined by masses, determined by 31%. In the DC analysis, a decrease in the spectroscopic signal was observed in both the far and near compared to the protein in solution (Figure 2A-B), which meant that the tertiary structure was altered with the formation of the Cu enzyme derivative. In fluorescence, the tryptophan fluorescence signal of the soluble enzyme exhibited a peak at 320 nm (Figure 2C) while in the hybrid a clear signal is observed at 385 nm, characteristic of the formation of a Cu²⁺ enzyme complex.

### NanoCu-2 and NanoCu-3

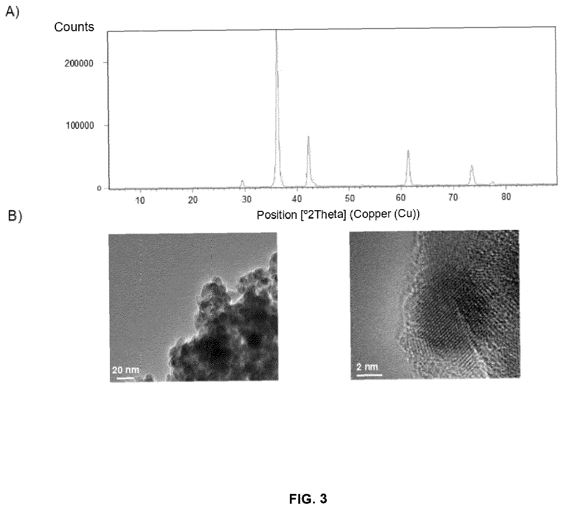

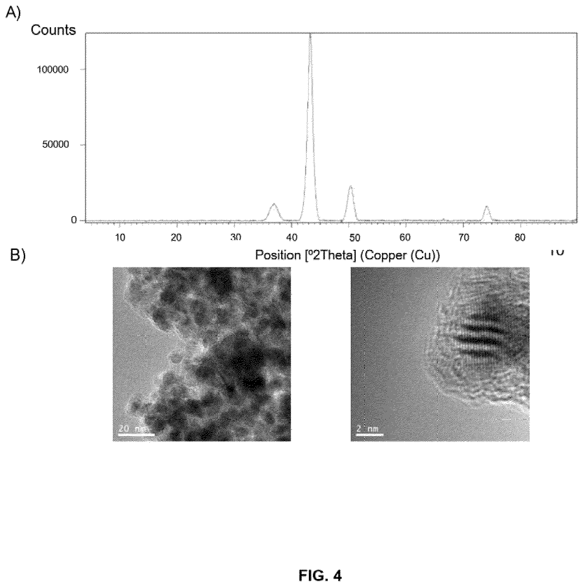

The XRD analysis showed that in the NanoCu-2 hybrid, the types of Cu was Cu(I), in the form of Cu₂O, exclusively (Figure 3). This biohybrid exhibited 61% Cu calculated by ICP analysis. TEM experiments revealed the formation of 10 nm nanoparticles (Figure 3B), In the case of the hybrid NanoCu-3, XRD showed that the main type was Cu(0) (80%), while the remainder was due to cuprous oxide (Figure 4). TEM analysis showed the formation of nanoparticles around 9 nm in size (Figure 4B). The ICP-OES showed that the Cu content in the NanoCu-3 biohybrid was 84% (The percentages of Cu in the hybrid material indicated in the present invention relate to percentages by weight).

### NanoCu-TLL, NanoCu-BTL, NanoCu-CAT, NanoCu-HSA

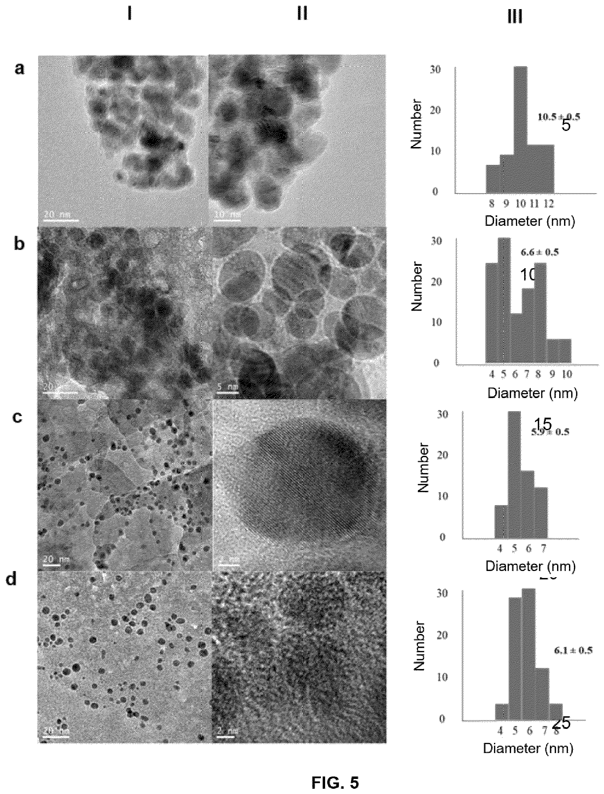

The new nanohybrids were structurally characterized by different techniques such as X-ray diffraction (XRD) (to determine their metallic type) and transmission electron microscopy (TEM) to determine the size of the copper nanoparticles. The XRD analysis showed that the type of copper was conversed in all cases, being Cu₃(PO₄)₂. TEM analysis showed the formation of nanoparticles of between 5.9 and 11 nm in diameter (Figure 5). The copper content of the material was determined by masses (ICP-OES), being around 32% in all cases.

### Example 3. Determination of the antiviral capacity of biohybrids against SARS-CoV-2

### NanoCu

To determine the antiviral activity of NanoCu against the SARS-CoV-2 virus, its action was studied against two virus proteins that act in two stages of the viral cycle.

One of the proteins is the 3CLpro protease involved in the replication of the virus within the human host cell. Inhibition of 3CLpro prevents the virus from multiplying inside the cell and thus blocks its infection process. 30 µg/ml NanoCu completely reduced the activity of 0.2 µM) protein after 20 min (Figure 6A). Assays were performed by continuously measuring FRET substrate hydrolysis in the presence of increasing concentrations of NanoCu and complete inhibition of 0.2 µM) protease activity was observed (Figure 6B). The determination of the inhibitory concentration, in which protease activity is reduced to 50% (IC50), corresponds to a concentration of 0.03 µM) Cu, while all activity was inhibited with a concentration of 0.1 µM) Cu (Figure 6C).

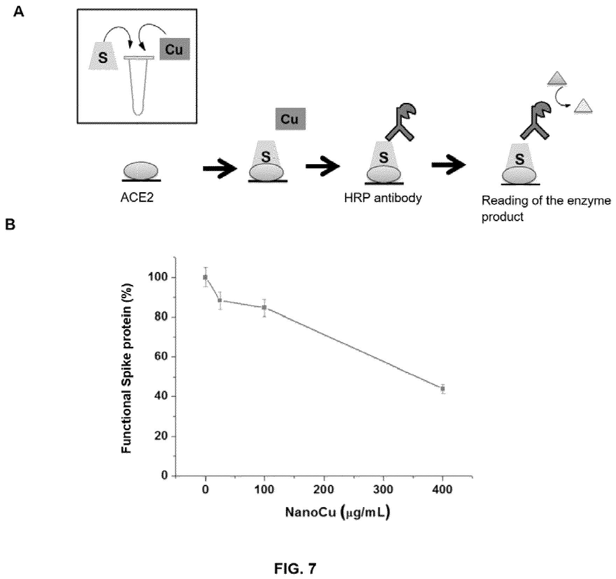

Another of the Spike proteins, is found in corona virus and its interaction with the ACE2 protein on the surface of human cells leads to the entry of the virus into the cell. Blocking the Spike-ACE2 interaction prevents the entry of the virus and therefore the infective capacity of the virus. The test that was performed is depicted in Figure 7A. The results obtained show that when the Spike protein interacts with NanoCu, its ability to interact with ACE2 decreased to 60% with only 400 µg/ml of NanoCu (Figure 7B).

### NanoCu-2 and NanoCu-3

The SARS-CoV-2 3CLpro protease inhibition results with these two NanoCu-2 hybrids (type is Cu₂O) and NanoCu-3 (type is Cu(0)) show that both types have inhibitory capacity similar to NanoCu (Figure 8).

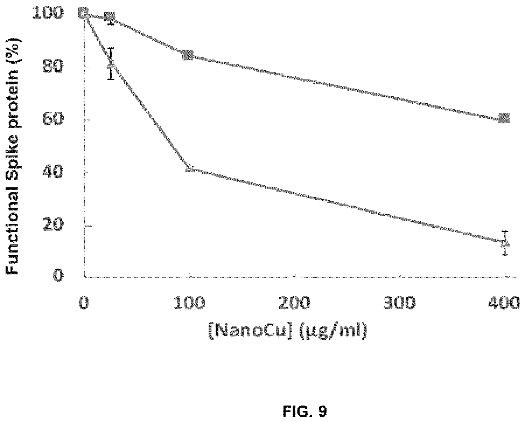

The effect of these two hybrids in blocking the Spike-ACE2 interaction was also evaluated. In Figure 9, it can be seen that NanoCu-2 (400 µg/ml) was slightly less effective than NanoCu, decreasing to 40% the capacity for interaction between S protein of the virus with ACE2. However, NanoCu-3 (Cu(0)) was more efficient in blocking the S protein, decreasing almost 90% with only 400 µg/ml of material.

### NanoCu-TLL, NanoCu-BTL, NanoCu-CAT, NanoCu-HSA

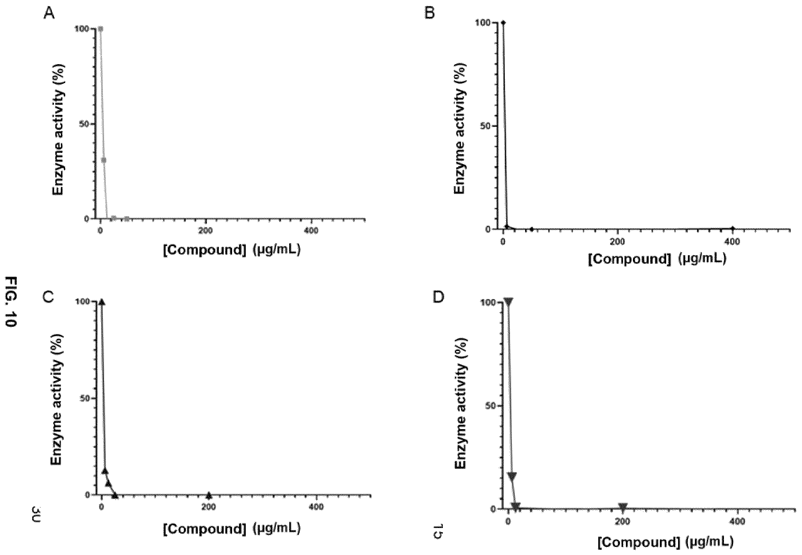

The results of inhibition of the SARS-CoV-2 3CLpro protease with the biohybrids prepared using other enzymes (TLL, BTL, HSA and CAT) yielded values similar to those obtained using CALB as the enzyme (Figure 10). This shows that this strategy can also be extended to the use of other proteins, with excellent ability to inhibit proteases due to Cu₃(PO₄)₂ nanoparticles between 4-11 nm synthesized and stabilized by the protein matrix.

### Example 4. Coating of the mask surface with NanoCu

For the direct application of NanoCu on the mask, 1 g of NanoCu was diluted in 800 µl of pure water and of different concentrations of ethanol:water (50:50, 70:30 and 80:20). By using a brush, the surface of a quarter of the mask was covered (45 cm²) homogeneously with subsequent drying by direct heat for 2 min.

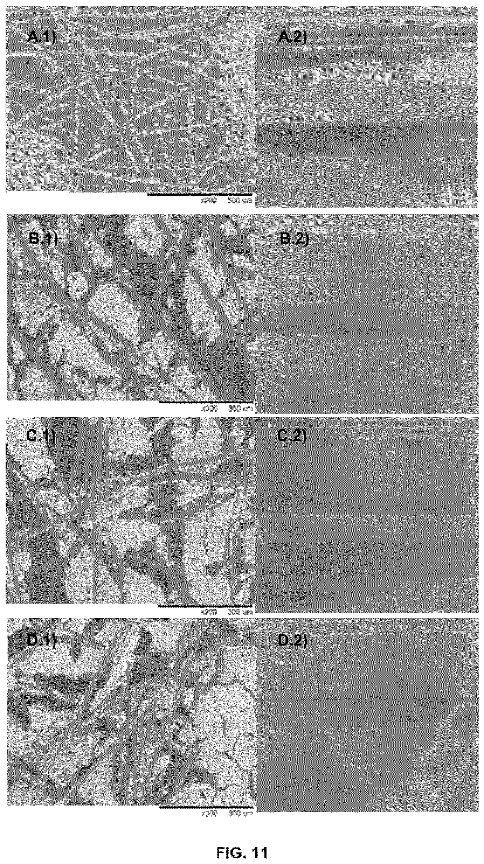

In Figure 11, the comparison with the different solvents is represented by SEM images, in which it is possible to observe how the material remains on the surface without appreciable differences. Furthermore, samples of real masks with the coating are shown, in which the surface coating can be seen. In this case, the 50:50 ethanol:water method was chosen for optimization.

To optimize the 50:50 ethanol:water coating method, a study was carried out with different concentrations of NanoCu, applying it with a brush and subsequent drying.

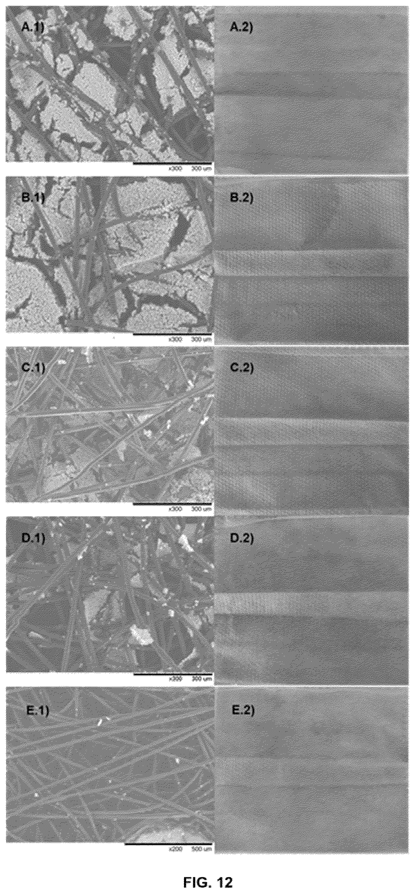

Through SEM images, the change in concentration can be observed directly (Figure 12). In this study, it is observed that there is a large change from 0.75 g to 0.5 g and between 0.5 g and 0.25 g. In all cases, a quarter mask was used, in other words, 45 cm² of surface. Thus, it has been observed that with the nanohybrid materials prepared in the present invention, the SARS-Cov-2 protease is inactivated using surprisingly low amounts.

### Example 5. Coating of 100% cotton fabric with NanoCu

For the direct application of NanoCu on the fabric, 0.25 g of NanoCu was diluted in 800 µl of ethanol:water 50:50. By using a brush, the entire 10.5 cm² surface of cloth was covered homogeneously with subsequent air drying.

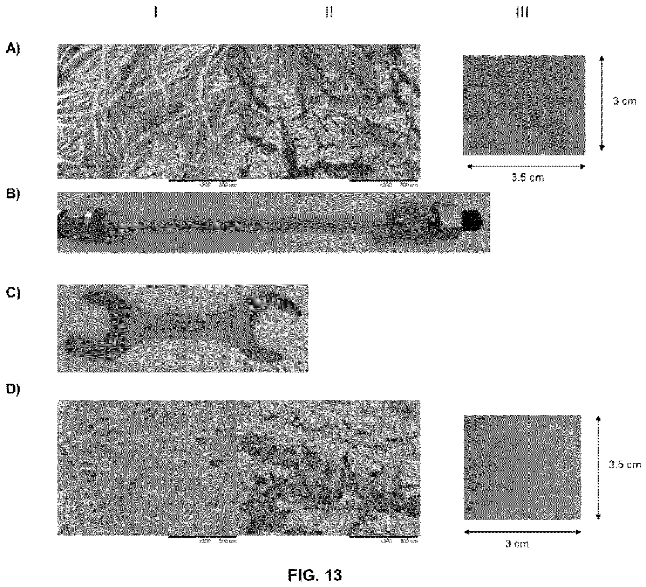

In Figure 13A, through SEM images, it can be observed how the fabric sample is before and after the adhesion of NanoCu on the surface.

### Example 6. Coating of the surface of metals (iron and steel) with NanoCu

For the direct application of NanoCu on metals, 0.25 g of non-lyophilized solid (or 25 mg of lyophilized hybrid) was diluted in 800 µl of 50:50 ethanol:water. By using a brush, the surface of 9 cm² was covered homogeneously (in the case of the iron wrench) and 6.9 cm² (in the case of the steel bar). Subsequently, it was left to air dry. Subsequently, 70% ethanol was applied to check if the coating came off the metal surface.

Figures 13B and 13C represent by means of a real image how the coating is distributed on the surface of the steel bar and on the iron wrench, respectively.

### Example 7. Coating of the paper surface with NanoCu

For the direct application of NanoCu on the paper (filter paper), 0.25 g of non-lyophilized solid (or 25 mg of lyophilized hybrid) was diluted in 800 µl of 50:50 ethanol:water. By using a brush, the 10.5 cm² surface was covered homogeneously with subsequent air drying.

In Figure 13D, through SEM images, it can be observed how the fabric sample is before and after the adhesion of NanoCu on the surface.

### Example 8. Stability of the NanoCu biohybrid

### Stability against contact with different disinfectants

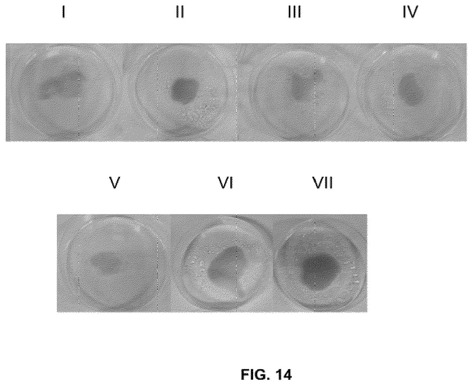

To evaluate the effect of the disinfectant on the stability of NanoCu previously adhered to a certain surface, samples of 0.25 g of solid were prepared with 800 µl of 50:50 ethanol:water which were each added to a Petri dish. After air drying (1-3 hours), 600 µl of each disinfectant was applied by spraying (Figure 14). The disinfectants used were 0.6% didecyldimethylammonium chloride (C₂₂H₄₈ClN) (commercial product Multipurpose Disinfectant, Green Forest), 3% hydrogen peroxide (obtained by diluting 33% hydrogen peroxide solution), 0.1% bleach (obtained by diluting commercial bleach (40 g sodium hypochlorite/L) in water), 70% and 96% ethanol, 70% isopropanol and 1.75% hexadecyltrimethylammonium bromide (CTAB). After air drying in a Petri dish, the solid was recovered. To verify that there was no change in the chemical type of the sample, they were analyzed by X-ray diffraction (XRD). In all cases, X-ray analysis of the samples of the treated material showed that there was no change in the chemical type of the sample in all cases.

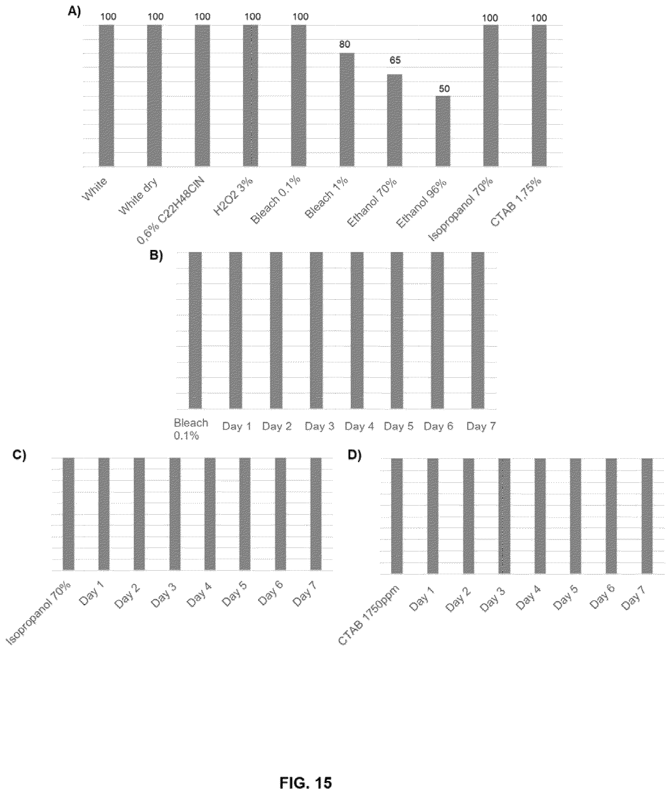

Furthermore, to evaluate the effect of the disinfectant in the deactivation of the catalytic activity of the Cu nanoparticles, solid samples were applied in the p-aminophenol (pAP) deamination reaction. There, it was observed how the catalytic efficiency of NanoCu was intact after treatment with the different disinfectants (Figure 15A).

### Stability against incubation in the presence of additives described as disinfectants

To evaluate the stability of NanoCu mixed directly with the disinfectant, 5 mg of the material were incubated in 7.5 ml of a solution (0.66 mg/ml) of 0.1% bleach, 70% isopropanol or 1.75% CTAB, evaluating its stability after 7 days of incubation. After that incubation time, the solids did not undergo any type of coloration or morphology change, the type of copper (demonstrated by X-rays) and the amount of copper (quantitative analysis) being perfectly preserved. Furthermore, it was confirmed that the catalytic efficiency of the nanoparticles in NanoCu was preserved at 100% after the incubation time (Figure 15B). This shows that this material could be further applied as a new additive for direct disinfection agent.

### High temperature stability

Another test carried out to assess the stability of NanoCu was at high temperature. In this case, the stability of the chemical structure was evaluated by exposing the material for 1 hour at 100°C in water and air, and at 150°C in an oil bath. The solids after the treatment were evaluated by means of X-ray diffraction. This certified that there were no changes in the chemical type of NanoCu, therefore it can be affirmed that the coating is stable at high temperature.

### Stability against washing in mask coatings

Once the mask has been coated with NanoCu, two washing experiments were performed, a wash at 25°C and 50°C with tap water (by placing the mask directly under the tap) to check that the surface coating did not come off for 3 cycles, and a second experiment incubating the mask in tap water at 40°C and 60°C for 2 hours to check its structural stability.

In the first experiment, a series of three washes was performed with running water at room temperature (25°C) and with hot running water (50°C). Samples were prepared with 0.5 g of solid in 800 µl of 50:50 ethanol:water (10.5 cm²). Between washes, the mask piece was allowed to air dry overnight.

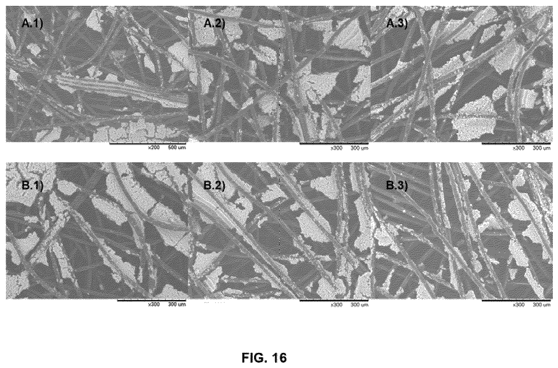

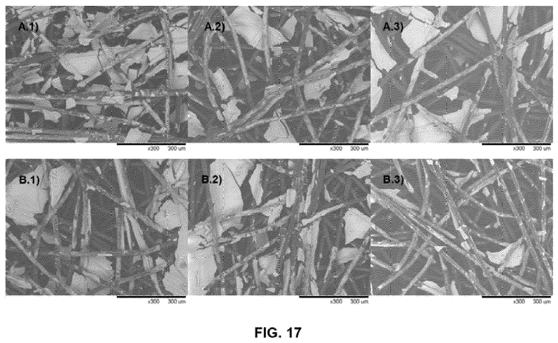

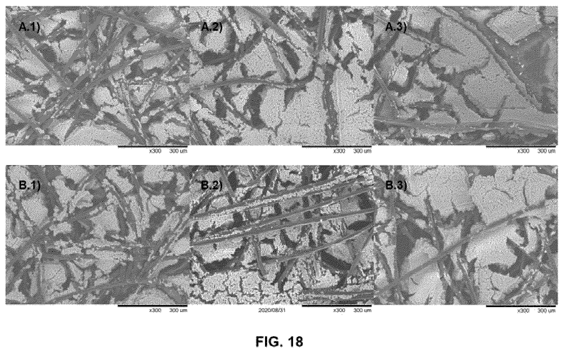

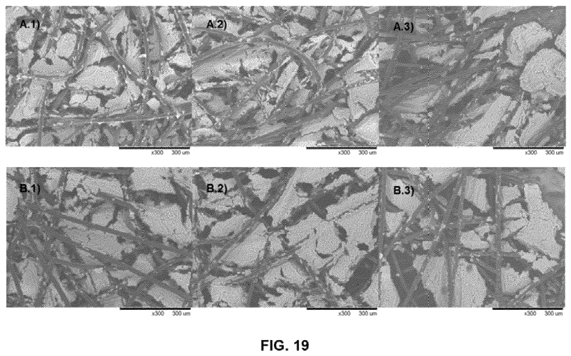

The effect on the NanoCu coating can be seen with the washes with different solvents (ethanol:water 50:50, 70:30, 80:20) (Figure 16-19). In any of the different experiments it can be observed that there are no changes in the washes. However, if we compare them with the SEM images of the initial samples (Figure 11), a slight change in the amount of NanoCu on the surface can be seen, especially with respect to those made with ethanol:water 70:30 and 80:20.

In the second experiment, a series of three washes (30 min, 1 hour and 2 hours) was carried out with running water at 40°C and 60°C. The samples were prepared with 0.12 g of solid in 800 µl of ethanol:water 50:50 (10.5 cm²). Once this had been carried out, the mask piece was allowed to air dry overnight.

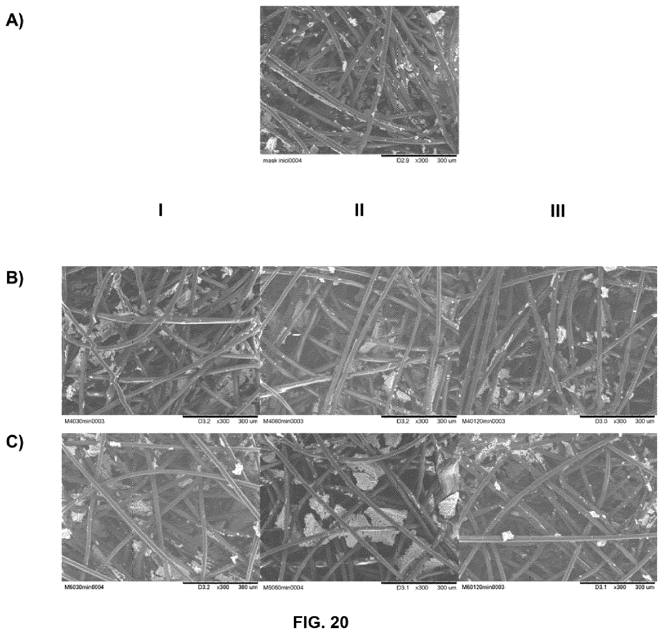

In any of the different experiments it can be observed that there are no changes in the washes with respect to the initial sample (Figure 20). To verify this fact, an elemental analysis of the samples was carried out and it was determined that there are no differences between them, therefore it is concluded that they are stable against washing.

### Stability against washing in coatings on 100% cotton fabric

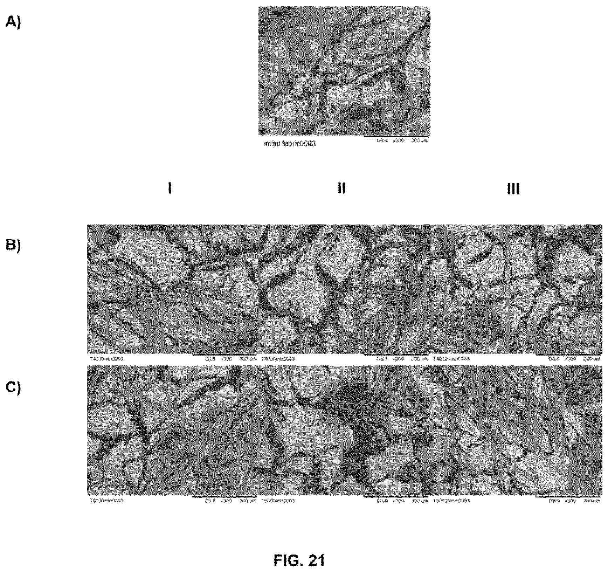

In the case of the fabric, it was incubated in tap water at 40°C and 60°C, performing a series of three washes (30 min, 1 hour and 2 hours). Once this had been carried out, the piece of fabric (containing 0.12 g of hybrid in 10.5 cm²) was allowed to air dry overnight. In any of the different experiments it can be observed that there are no changes after the washes with respect to the initial sample (Figure 21). To verify this fact, the samples were analyzed in chemical analysis and it was determined that there are no differences between them, therefore it is concluded that they are stable against washing.

### Stability against washing in coatings of metals (Steel and iron)

In the case of metals, a wash was carried out by adding ethanol:water 70:30 to verify that the coating did not come off the surface, resulting in non-desorption of the coating.

### Example 9. Catalytic efficiency of NanoCu biohybrid determined by p-aminophenol dehydroxylation reaction

The catalytic efficiency of NanoCu was determined using the p-aminophenol hydroxylation reaction as a model reaction. 1 mg of p-aminophenol was dissolved in 10 ml of distilled water and 100 mM hydrogen peroxide (1%, v/v). To start the reaction, 3 mg of NanoCu (without or with different treatments) were added and gently stirred at room temperature in an orbital agitator (320 rpm). The reaction was followed by colorimetric determination (color appearance) by means of ultraviolet spectrometry at a wavelength of 270 nm at a reaction time of 3 min.

### Example 10. Study of the antimicrobial activity of NanoCu

Microbial activity was determined by the agar disk diffusion method using two bacteria, *Escherichia coli* (gram negative) and *Bacillus subtilis* (gram positive) from the Spanish culture type collection (CECT).

### 1. Luria-Bertani (LB) culture broth preparation protocol:

10 g/l tryptone was mixed with 5 g/l sodium chloride (NaCl) and 5 g/l yeast in distilled water. Once mixed, 13.5 g of agar-agar was added to 900 ml of LB broth. The plates were prepared with the culture broth with agar-agar, and once solidified, an antibiotic was added, specifically, 100 µl of ampicillin (100 mg/ml) per plate.

### 2. Preparation protocol of the bacteria strains:

The remaining 100 ml of the culture broth were used for the preparation of the strains. For each strain, 200 µl of each strain were inoculated into the LB broth in a 250 ml Erlenmeyer flask and with orbital stirring at 150 rpm at 37°C overnight. Subsequently, the absorbance at 600 nm was measured, obtaining the value of the optical density of 2 for *B. subtilis* and 4 for *E.coli.*

### 3. Agar Disk Diffusion Assay:

After preparing the culture plates, the strains were seeded on the plates. 100 µl of the colony of *E.coli or B.subtilis* for each plate, they were added to the entire surface of the agar medium with the help of the "cepeda" sowing loop, guaranteeing a homogeneous pre-inoculation. Next, the Whatmam 9 mm filter paper disc was impregnated with 25 µl of the corresponding sample, allowing it to dry for 3-4 min and placing it on the surface of the plate previously pre-inoculated with the colony. The plates were incubated in an inverted oven at 37°C for 24 hours and then the inhibition zones were measured, including the diameter of the discs. Blanks were made as control samples for the analyzes.

Next, the samples analyzed were diluted to different concentrations starting from a concentration of 5000 ppm. The concentrations studied were 5000 ppm, 2500 ppm, 1250 ppm, 650 ppm, 500 ppm, 250 ppm and a control sample (water).

The antimicrobial activity results of NanoCu with these two strains (*E.coli* and *B. subtilis*) show that it has an inhibitory capacity up to 500 ppm for *E.coli* and up to 625 ppm for *B*. *subtilis* (Table 1).

**Table 1. Study of the sensitivity of NanoCu against bacteria**

| | ***E.coli*** | ***B. subtilis*** |
|---|---|---|
| **Sample (ppm)** | **Inhibition zone (mm)** | **Inhibition zone (mm)** |
| Control | 0 | 0 |
| 5000 | 32 | 22 |
| 2500 | 25 | 22 |
| 1250 | 20 | 20 |
| 625 | 18 | 12 |
| 500 | 12 | 0 |
| 250 | 0 | 0 |

## Claims

1. A non-therapeutic use of a hybrid material comprising:
- a protein matrix composed of an enzyme or protein, and
- spherical nanoparticles of types of copper independently selected from Cu₃(PO₄)₂, Cu₂O and Cu (0), where said nanoparticles are distributed in a monodisperse manner within the matrix,
as a biocidal agent.

2. The use according to the preceding claim, where the protein matrix is selected from *Candida antarctica* lipase B, *Thermomyces lanuginosus* lipase, *Bacillus thermocatenulatus* lipase, human albumin protein and catalase.

3. The use according to any of the preceding claims, where the nanoparticles are Cu₃(PO₄)₂ and exhibit a single diameter size distribution of between 3±0.5 nm and 15±0.5 nm.

4. The use according to any of the preceding claims, where the single diameter size distribution is of between 3±0.5 nm and 5±0.5 nm.

5. The use according to any of the preceding claims, where the biocidal agent is used against a virus selected from the hepatitis C virus, SARS-CoV, MERS-CoV, and SARS-CoV-2 that causes Covid-19.

6. The use according to any of claims 1 to 4, where the biocidal agent is used against a bacterium selected from *Escherichia coli* and *Bacillus subtilis.*

7. The use according to any of the preceding claims, where the hybrid material acts as a coating agent.

8. The use according to the preceding claim, where the coating is applied on surfaces of materials selected from ceramic materials, polymeric materials, metals, paper, textile and masks.

9. The use according to the preceding claim, where the metal is iron or steel.

10. The use according to claim 8, where the textile material is cotton, preferably 100% cotton.

11. The use according to any of claims 7 to 10, where the coating agent is used as an additive for direct disinfection.

12. The use according to the preceding claim, where the agent is added to a solution selected from sodium hypochlorite, hydrogen peroxide, isopropanol, ethanol, didecyldimethylammonium chloride, and hexadecyltrimethylammonium bromide.

13. A composite material comprising:
a. a product composed of a material selected from ceramic material, polymeric material, metal, paper, textile or a mask; and
b. a coating comprising the hybrid material as described in claims 1 to 4.

14. The material according to the preceding claim, where the metal is iron or steel.

15. The material according to claim 13, where the textile material is cotton, preferably 100% cotton.

16. The material according to claim 13, where the material is a mask.

17. A method for obtaining the composite material according to any of claims 13 to 16, which comprises applying the coating dissolved in a hydroalcoholic solution on the surface of a product composed of ceramic material, polymeric material, metal, paper, textile or a mask.

18. The method according to the preceding claim, where the coating is applied dissolved in a hydroalcoholic solution with an alcohol:water concentration of between 50:50 and 80:20.

19. The method according to the preceding claim, where the alcohol is selected from ethanol, propanol, isopropanol, and methanol.

20. The method according to the preceding claim, where the concentration of the alcohol:water mixture is 50:50, and alcohol is ethanol.

21. A hybrid material comprising:
- a protein matrix composed of an enzyme or protein selected from *Thermomyces lanuginosus* lipase, *Bacillus thermocatenulatus* lipase, human albumin protein and catalase, and
- spherical nanoparticles of Cu₃(PO₄)₂, where said nanoparticles are distributed in a monodisperse manner within the matrix and have a single diameter size distribution of between 3±0.5 nm and 15±0.5 nm.

22. A method for obtaining the hybrid material described according to claim 20 **characterized in that** it comprises the following steps:
a) addition under stirring of an enzyme or protein selected from *Thermomyces lanuginosus* lipase, *Bacillus thermocatenulatus* lipase, human albumin protein and catalase, that form the protein matrix to a phosphate buffer solution at pH 7;
b) addition of a copper salt to the solution obtained in step a) at room temperature of between 20 and 25°C;
c) incubation of the solution obtained in step b) for a time between 14-20 hours; and
d) collecting and washing the hybrid material obtained in the previous step,
e) optionally drying the product obtained from step (d).

23. The method according to the preceding claim, in which the hybrid material collected in step (d) is dried by lyophilization in step e).

24. The method according to any of claims 22 to 23, where the copper salt in step (b) is Cu₂SO₄·5H₂O.

25. The method according to any of claims 22 to 24, wherein 10 mg of copper salt are added in step b) per ml of buffer solution.

26. A disinfectant solution comprising the hybrid material according to claim 21 dissolved in a medium selected from sodium hypochlorite, hydrogen peroxide, isopropanol, ethanol, didecyldimethylammonium chloride, and hexadecyltrimethylammonium bromide.
